# EUROPEAN PATENT SPECIFICATION

(11) **EP 3 169 322 B1**
(45) Date of publication and mention of the grant of the patent: **09.09.2020**
(21) Application number: 15754018.8
(22) Date of filing: 17.07.2015
(51) Int. Cl.: A61K 31/366, A61K 31/045, A61K 31/575, A61K 31/455, A61K 45/06, A61K 36/062, A61K 9/28, A61K 9/68, A61K 9/48, A61K 9/00, A61K 9/10, A61P 3/06, A61K 36/899, A61K 47/26, A61K 9/20

(54) **DIETETIC COMPOSITION WITH ANTIDYSLIPIDEMIC ACTIVITY**
DIÄTETISCHE ZUSAMMENSETZUNG MIT ANTIDYSLIPIDÄMISCHER AKTIVITÄT
COMPOSITION DIÉTÉTIQUE PRÉSENTANT UNE ACTIVITÉ ANTIDYSLIPIDÉMIQUE

(30) Priority: 18.07.2014 IT RM20140401
(43) Date of publication of application: 24.05.2017
(73) Proprietor: BERLIN-CHEMIE Aktiengesellschaft, 12489 Berlin (DE)
(72) Inventor: MELANI, Francesco, I-50014 Fiesole (FI) (IT); AGOSTINI, Alida, I-47865 San Leo (RN) (IT)
(74) Representative: Di Giovine, Paolo
(86) International application number: PCT/IB2015/055430
(87) International publication number: WO 2016/009403

(56) References cited:
- US-A1- 2004 033 202
- US-A1- 2012 177 623
- CLAUDIA STEFANUTTI ET AL: "Combined Treatment with Dif1stat and Diet Reduce Plasma Lipid Indicators of Moderate Hypercholesterolemia More Effectively than Diet Alone: A Randomized Trial in Parallel Groups", LIPIDS, vol. 44, no. 12, 1 December 2009 (2009-12-01), pages 1141-1148, XP055136060, ISSN: 0024-4201, DOI: 10.1007/s11745-009-3368-5
- CICERO ET AL: "Antihyperlipidaemic effect of a Monascus purpureus brand dietary supplement on a large sample of subjects at low risk for cardiovascular disease: A pilot study", COMPLEMENTARY THERAPIES IN MEDICINE, CHURCHILL LIVINGSTONE, EDINBURGH, GB, vol. 13, no. 4, 1 December 2005 (2005-12-01), pages 273-278, XP005198466, ISSN: 0965-2299, DOI: 10.1016/J.CTIM.2005.07.008
- KIRSI LAITINEN ET AL: "Dose-dependent LDL-cholesterol lowering effect by plant stanol ester consumption: clinical evidence", LIPIDS IN HEALTH AND DISEASE, BIOMED CENTRAL, LONDON, GB, vol. 11, no. 1, 22 October 2012 (2012-10-22), page 140, XP021136364, ISSN: 1476-511X, DOI: 10.1186/1476-511X-11-140

## Description

### STATE OF ART

Dyslipidemia, that is the alteration of the fat values in blood and, more particularly, hypercholesterolemia, and that is an excess of cholesterol in blood, is considered one of the five main risk factors, together with hypertension, diebetes mellitus, smoke and obesity, for the onset of a whole series of cardiovascular pathologies typical of the so-called "welfare society", thereamong atherosclerosis and the diseases connected thereto, thereamong, for example, angina pectoris, heart attack and stroke.

Recent data reported in literature (European Cardiovascular Disease Statistics 2012 Edition) show in more than 4 millions each year the deaths due to cardiovascular pathologies in Europe, corresponding to almost half (47%) of the total deaths. In the same document the direct correlation between the levels of plasmatic cholesterol and the risk of the onset of cardiovascular pathologies is confirmed by a series of experimental data. The World Health Report 2002 estimates that about 60% of cardiovascular diseases and about 40% of anemic infarcts in the developed countries are due to the levels of exceeding total cholesterol with respect to the theoretical minimum level (3.8 mmol/L).

In order to reduce the risk factor correlated to dyslipidemias, pharmacological treatements have been available for a long time, mainly based upon the administration of active principles belonging to the group of statins, such as for example mevinolin otherwise known as lovastatin or monakolin K.

Such active principles selectively inhibit the enzyme of hydroxymetyl-glutaryl-coenzyme A reductase (HMG-CoA- reductase), a hepatic enzyme allowing the biosynthesis of cholesterol.

Although the pharmacological therapy based upon statins allows reducing the endogenous cholesterol from 15 to 55% and has an undoubt effectiveness in the treatment of dyslipidemias, at the same time it involves the not so frequent onset of unwished side effects which often appear with an alteration of the liver functionality of liver, which is the organ responsible for the metabolism of the various involved molecules.

In a not negligible percentage of the pharmacologically treated patients, in fact, an alteration appears in the enzymes correlated to the liver and myocardium metabolism, such as for example the so-called transaminases (glutamic oxaloacetic transaminase or GOT or the glutamic-pyruvic transaminase or GPT) or in the enzymes involved in the operation of the skeletal muscle tissue such as for example creatine phosphokinase (CPK).

In addition to this, the pharmacological treatments have rather high costs both for the patient and for the health system.

In order to avoid the drawbacks of the pharmacological treatments, alternative routes for the treatment of dyslipidemia have then been studied and scientifically evaluated, mainly based upon dietetic compositions and food supplements based upon natural nutrients.

Several natural compounds, known for a long time for their antidyslipidemic action, have been clinically evaluated by determining, when possible, the probable mechanism implied by such activity. Among these substances we mention: niacin which inhibits the oxydation of LDL-cholesterol and reduces the lipoprotein with low density and the triglycerides; red yeast rice, a product obtained from fermentation of rice thanks to a yeast *(Monascus ruber* or *Monascus purpureus)* containing, apart from sterols, isoflavones and other compounds, monacolin K, a statin inhibiting the cholesterol synthesis via HMG-CoA reductase; the long chain linear aliphatic alcohols such as for example the polycosanols commonly extracted from the sugar cane; the sterols of vegetable origin or phytosterols and the corresponding saturated derivatives, stanols, reducing the absorption of cholesterol at intestinal level (exogenous cholesterol); some mono-unsaturated fatty acids contained in olives, olive oil and in fruits with shell reducing the level of LDL and triglycerides; soya bean which shows a moderate reduction in the levels of total cholesterol, of triglycerides and of LDL; the green tea and the extracts thereof containing catechins which reduce the absorption of cholesterol at intestinal level and the levels of triglycerides; the fatty acids of the Omega-3 series therefor epidemiologic observations and clinical studies have shown significative reduction in triglycerides, number of particles of LDL and VLDL; the garlic which reduces the level of total cholesterol and of LDL), sesame, guggul *(Commiphora mukul)*, resveratrol, curcumin, pantetin and others (Journal of Clinical Hyperthension 2012, 14, 121 - Official Journal of the American Society of Hyperthension).

Although there is a series of clinical works demonstrating the hypocholesterolemizing activity of long chain linear aliphatic alcohols, the action mechanism thereof still results not completely clear. The results of some clinical studies would seem to show an action inhibiting the cholesterol synthetis, an increase in the absorption of LDL at hepatic level and of the catabolism of the same (American Hearth Journal 2002, 143, 356).

Niacin, under the form of coenzymes (nicotin adenin dinucleotide or NAD and nicotin adenin dinucleotide phosphato or NADP) takes part in several oxydoreduction reactions, at level of both catabolic and anabolic processes, such as the synthesis of fatty acids and aminoacids. Several controlled clinical studies have shown a significative reduction in the cardiovascular events after administration of niacin (Mini Reviews in Medicinal Chem 2010, 10, 204) and it is recommended, alone or in combination with other active agents, in the treatment of dyslipidemia (National Institute of Health (NHI); National Hearth Lung and Blood Institute; 2002 National Cholesterol Education Program (NCEP) - Adult Treatment Panel III - NHI Publication n° 02-5212, Sept 2002). Niacin showed to have a correlated dose effect in the reduction in the level of total cholesterol, of LDL, of the number of particles of LDL, of triglycerides and of apolipoprotein B and in the increase in the level of HDL (Progr. Cardiovasc. Disease 2009, 52, 61), and the recommended effective dose varies from 500 to 4000 mg per day.

As far as the phytosterols in particular is concerned, the European Food Safety Authority (EFSA) has recently issued a scientific opinion wherein the existance of a dose-dependant correlation between the consumption of vegetable sterols and stanols and the reduction in the level of cholesterol in blood is admitted (EFSA Journal 2010, 8, 1838). In the document it is further concluded that a daily consumption of an amount equal to 1.5-2.4 g of vegetable sterols and/or stanols can allow a clinically significative reduction in the levels of LDL-cholesterol between 7 and 10%. The ingestion of higher amounts of phytosterols, however, does not lead to a significative corresponding higher reduction in the levels of cholesterol in the treated subjects. Furthermore, no interaction between drugs and nutrients has been detected upon using a diet enriched with sterols and/or stanols, by making wholly safe the use thereof.

According to the previous evaluation, a recent meta-analisys performed by European Atherosclerosis Society (Gylling H et al: Plant sterols and plant stanols in the management of dyslipidaemia and prevention of cardiovascolar disease, Atherosclerosis (2013), doi: 10.1016/j.atherosclerosis.2013.11.043) shows that the consumption of food enriched with vegetable sterols/stanols (2 g/per day) causes a significant reduction in the level of LDC-cholesterol by a value comprised between 8 and 10%. Moreover, with the dose of 2 g per day the reduction of LDL-cholesterol is additive to that with statins in dyslipidemic subjects and equivalent or greater than that obtained with the double dose of statins. Furthermore, although they deserve additional deeper investigation, data are available showing a reduction in the level of triglycerides by a value of 6-9% upon the ingestion of 1.5-2 g per day of vegetable sterols/stanols. Although the recommended consumption of sterols/stanols around 2g per day can be considered safe, however in some clinical studies a reduction in the levels of carotenoids has been found, above all the more lipophilic ones such as beta-carotene or licopene, probably due to a reduced intestinal absorption (Gylling H et al, Atherosclerosis (2013), page 22).

Various compositions based upon natural nutrients have been reported in literature for the treatment of dyslipidemia. In particular WO2013023826 describes a food composition useful for the decrease in the plasmatic cholesterol comprising an aqueous phase and a fat phase with 0.1 - 20% of phytosterols, corresponding to at least 0.3g of phytosterols per day; WO2008049196 describes a dietetic integrator to be administered twice a day comprising 0.3-1.1 g of sterol or sterol derivativatives of vegetable origin, 1-90 mg of procyanidin, 0.5-7.5 mg of polycosanols, and 10-70 mg of niacin capable of improving the level of plasmatic cholesterol by decreasing the level of LDL-cholesterol, by increasing the level of HDL and by interfering with the activity of the HMG-CoA enzyme involved in the synthesis of the cholesterol itself. US2012/0270849 describes a composition containing niacin or derivatives thereof, Omega-3 fatty acids, and phytosterols effective in the adjustment of the levels of both HDL and LDL cholesterol and of triglycerides.

IT1347883 shows a composition of nutraceutics comprising fermented red rice, niacin and polycosanols capable of reducing the level of total cholesterol up to a value of 24%. In particular IT1347883 describes compositions including for each single dose: a) dry extract of Monascus purpureus between 400 and 600 mg equal to a content of mevinolin between 1.2 and 2.4 mg, b) polycosanols from sugar cane wax between 2 and 2.8 mg with a content in octacosanol between 0.6 and 2.0 mg, c) niacin between 9 and 15 mg. In the described compositions it is also provided the presence of other components as coadiuvants and stimulants of the metabolism such as vitamin E, the folic acid, vitamin B6 and vitamin B12.

Stefanutti C. et al. LIPIDS, vol. 44, no. 12, pages 1141-1148 (2009) and Cicero et al. COMPLEMENTARY THERAPIES IN MEDICINE, vol. 13, n. 4, pages 273-278 (2005) both disclose a composition with antilipidemic activity comprising only red yeast rice, policosanols and niacin.

Although the dietetic compositions based upon natural nutrients are capable of obtaining encouraging results against a substantial absence of unwished side effects, the effectiveness of these compositions, however, still results to be far from that of the conventional drugs.

### SUMMARY OF THE INVENTION

The invention is as defined in the appended claims.

The present invention is directed to a composition of natural nutrients comprising for each unit dosage, to be administered preferably once a day, an effective amount of: a fermented rice by means of a yeast selected among the red yeasts of rice (red yeast rice), polycosanols, niacin, sterols or stanols and suitable excipients.

The subject of the present application are then compositions as described in claims 1 to 16.

The composition according to the present invention has shown an unexpected increase in the antidyslipidemic activity, higher than what expected based upon the activity performed by each component, without causing any unwished side effect. Therefore a second subject of the present application are said compositions to be used in the treatment or prophylaxis of dyslipidemias.

Furthermore with the amounts of sterols/stanols used by us it has been possible, with the other active principles and the suitable excipients, implementing pharmaceutically stable compositions, suitable to an industrial manufacturing, and suitable to an acceptable administration.

### DETAILED DESCRIPTION OF THE INVENTION

The composition implemented according to the present invention has been surprisingly capable of obtaining an unexpected increased in the antidyslipidemic activity without inducing any undesired side effect, such as for example metabolic or enzymatic alterations on the organism, by using at the same time some ingredients with antidyslipidemic action in lower amounts than those generally provided in dietetic compositions of the known art.

What said has been possible above all by dosing the used amount of sterols/stanols.

The use of 1-3 g/die of stanols/sterols as generally recommended can involve a bad absorption of vitamin and of carotenoids in general. Moreover, the use of large amounts of liposoluble compounds, such as indeed sterols/stanols to be mixed with the other components of the composition, can constitute a problem not easy to be solved as far as the manufacturing of a pharmaceutical composition suitable to the patient's expectations is concerned. Surprisingly it has now been found that by using an amount of stanols/sterols between 0.5 and 1g for single daily dose, in association to the other components of the composition, it is possible keeping a reduction in the levels of LDL-cholesterol not lower to what would be obtainable with analogous use of sterols/stanols in the generally recommended amounts. We have had then evident indications that a reduced dosage of sterols/stanols, when associated to the other components of the composition, does not cause the predictable effectiveness reduction in decreasing the levels of LDL-cholesterol with respect to the dosages of sterols/stanols generally recommended when analogously associated to the same components.

The use of the compositions according to the present invention in subjects with hypocholesterolaemia in fact has involved a reduction in the levels of total cholesterol up to 27% already after 2 months as from the treatment beginning.

The composition of the invention, then, can be used in a particularly advantageous and preferred way in the treatment or prophylaxis of dyslipidemias. In particular the above-mentioned composition allows using a lower amount of vegetable sterols than that indicated as effective in the recent guidelines (US Third Report of the National Cholesterol Education Program on Detection Evaluation and Treatment of High Blood Cholesterol in Adults) (Adult treatment panel III) and those issued by the American Heart Association.

More in particular, it has been found that the above-mentioned association of ingredients shows an unexpected synergic effect and it is able to obtain a high reduction in the cholesterol level even with lower dosages of sterols than those generally recommended in the state of art.

Therefore, the compositions of the invention are administered according to a dosage regime providing daily administrations of sterols/stanols from 500 to 2000 mg/die, preferably from 500 to 1000 mg/die, for example about 800 mg/die.

In some cases, the treatment can provide an initial phase with daily administrations of sterols/stanols from 500 to 2000 mg/die followed by a maintenance phase with daily administrations from 500 to 1000 mg/die.

According to the pathology gravity, the treatment can be continued for a period from 2 to 12 months, or from 1 to 5 years or in chronic form for the whole life.

The compositions of the invention include, per dosage unit, apart from at least one pharmaceutically acceptable excipient, the four components in the following independent amounts.
- red yeast rice having a titre in mevinolin comprised between 0.05 and 5% by weight;
- polycosanols;
- niacin; -
- from 500 to 1000 mg, preferably from 600 to 900 mg of sterols and/or stanols or
- red yeast rice having a titre in mevinolin comprised between 0.05 and 5% by weight;
- polycosanols;
- from 4 to 100 mg, preferably from 10 to 50 mg of niacin; and
- from 500 to 1000 mg, preferably from 600 to 900 mg of sterols and/or stanols or
- red yeast rice having a titre in mevinolin comprised between 0.05 and 5% by weight;
- from 1 to 50 mg, preferably from 5 to 20 mg, of polycosanols;
- niacin; and from 500 to 1000 mg, preferably from 600 to 900 mg of sterols and/or stanols or
- from 50 to 600 mg, preferably from 100 to 300 mg, of red yeast rice having a titre in mevinolin comprised between 0.05 and 5% by weight;
- polycosanols;
- niacin; and
- from 500 to 1000 mg, preferably from 600 to 900 mg of sterols and/or stanols

Within the present invention, it has also been found that it is possible obtaining an antidyslipidemic effect with particular effectiveness when the various ingredients of the composition are present in specific ponderal relationships one with respect to the other one. Generally, for the objects of the invention, the composition comprises for each unit dosage:
- from 50 to 600 mg of dry extract of a rice ferment thanks to a yeast selected among the red yeast rice, said extract having a titre in mevinolin comprised between 0.05 and 5% by weight;
- from 1 to 50 mg of polycosanols;
- from 4 to 100 mg of niacin; and
- from 500 to 1000 mg from sterols and/or stanols
- at least one pharmaceutically acceptable excipient

In this way, it has resulted to be possible introducing the needed amounts of active ingredients from the point of view of a reduction in the dyslipidemias by obtaining the above-mentioned unexpected and advantageous synergic effect.

A preferred embodiment of the present invention is constituted by a composition including:
- from 100 to 300 mg of dry extract of a rice ferment thanks to a yeast selected among the red yeast rice, said extract having a titre in mevinolin comprised between 0.05 and 5% by weight
- from 5 to 20 mg of polycosanols;
- from 10 to 50 mg of niacin; and
- from 600 to 900 mg from sterols and/or stanols.
- at least one pharmaceutically acceptable excipient.

Examples of compositions according to the invention comprise, apart from a suitable excipient:
- 167 mg of dry extract of a rice ferment thanks to a red yeast rice
- 10 mg of polycosanols;
- 35 mg of niacin; and
- 800 mg from sterols and/or stanols.
or:
- 168 mg of dry extract of a rice ferment thanks to a red yeast rice;
- 10 mg of polycosanols;
- 27 mg of niacin; and
- 890 mg from sterols and/or stanols (with a titre of 90%).
or:
- 167 mg of dry extract of a rice ferment thanks to a red yeast rice;
- 10 mg of polycosanols;
- 32 mg of niacin; and
- 800 mg from sterols and/or stanols.

Moreover, in the composition there can be other vitamins selected among the group constituted by: folic acid and its derivatives, vitamin B6, vitamin B12 and vitamin E.

### Red yeasts from rice

One of the ingredients with antidyslipidemic action of the composition of the invention is constituted by a rice ferment by a yeast selected among the red yeast rice.

Preferably, the rise ferment by a yeast is used in the composition of the invention in the form of dry extract available in commerce with various titres (or standards) in mevinolin (lovastatin or monakolin K). For the objects of the invention, the titre in mevinolin of the dry extract is comprised between 0.05 and 5% by weight and, preferably, between 1 and 4% by weight.

Preferably, the composition of the invention comprises for each daily dosed amount an amount of the above-mentioned rice ferment (as dry extract) so as to deliver a total amount of mevinolin comprised between 0.5 and 15 mg.

More preferably, the amount of said rice ferment is so as to deliver a total amount of mevinolin comprised between 1 and 10 mg and still more preferably comprised between 2 and 6 mg.

In such case, in fact, it has been found that the amount of active principle (mevinolin) introduced into the organism is such as to obtain the greatest effectiveness while achieving at the same time the above-mentioned surprisingly synergic effect when in combination with the remaining antidyslipidemic ingredients (aliphatic alcohol, niacin and phytosterols).

To this regard, it is further to be noted that in the composition of the invention the above active principle (mevinolin) is in the form of complex with oligosaccharides and other substances, which generally increase the bioavailability thereof by contributing to reduce drastically the side effects thereof. These side effects can be considered as substantially absent compared to the administration of the same isolated and purified active principle typical of the pharmacological-type treatments. Preferably, the yeast used in the rice fermentation for the purpose of the invention is a yeast belonging to the class *Ascomycota* order *Eurotiales,* genus *Basipetospora,* species *Monascus.* Still more preferably, such microrganism is a yeast *Monascus purpureus* or *Monascus ruber* and the obtainable dry extract of rice ferment generally can be found in commerce with titres (or standards) in mevinolin comprised between the above-mentioned values of 0.05 and 5% by weight.

For the purpose of the present invention such dry extract of rice ferment obtained by a yeast is commonly defined as 'red yeast rice'.

### Linear aliphatic alcohol

A second ingredient among the ones with antidyslipidemic action of the composition of the invention is constituted by a mixture of linear aliphatic alcohols having from 14 to 36 carbon atoms, otherwise known with the term of "polycosanols".

Preferably, the above-mentioned mixture of linear aliphatic alcohols has a titre in octacosanol, a linear alcohol having 28 carbon atoms, comprised between 20 and 80% by weight.

The above-mentioned mixture of linear aliphatic alcohols can be obtained from a series of natural sources, thereamong it is possible mentioning - for example - wheat germ wax, rice germ wax, carnauba wax (exudate of leaves of *Copernicia prunifera*), sugar cane wax, beeswax, avocado oil, alfaalfa, bamboo and apple wax. Depending upon the specific origin of the mixture of linear aliphatic alcohols, the titre in octacosanol preferably can be comprised between 14 and 22% in polycosanols from beeswax, between 50 and 60% in the supercritic extract of polycosanols from rice wax and between 50 and 80% in polycosanols from sugar cane wax.

Preferably, the above-mentioned mixture of linear aliphatic alcohols comprises, apart from octacosanol even tetracosanol (alcohol with 24 carbon atoms), exacosanol (alcohol with 26 carbon atoms), triacontanol (alcohol with 30 carbon atoms) and dotriacontanol (alcohol with 32 carbon atoms).

In an additional preferred embodiment the composition of the invention then comprises polycosanols consisting of a mixture of linear aliphatic alcohols having 24 to 32 carbon atoms.

### Niacin

A third ingredient among the ones with antidyslipidemic action of the composition of the invention is constituted by niacin also known as vitamin B3 or nitotinic acid.

Within the present description and in the subsequent claims, under the term of niacin, one wants to designate both the nicotinic acid as such and the amide thereof, nicotinamide.

In an aspect of the present invention the composition of nutrients comprises for each daily dosed amount an amount of niacin comprised between 4 and 100 mg. Preferably, the composition of the invention comprises for each daily dosed amount an amount of niacin comprised between 10 and 50 mg.

### Sterols or stanols

A fourth ingredient among the ones of the composition of the invention is constituted by sterols or stanols. The vegetable sterols are steroid alkaloids differing from cholesterol in the structure of the side chain thereof, whereas the vegetable stanols are 5α-saturated derivatives of the corresponding sterols. The main sources of vegetable sterols are vegetable oils, seeds, cereals, fruits with shell and vegetable ones (beans, mais, black olives, cauliflowers, oranges). The most abundant phytosterols are sitosterol, campesterol and stigmasterol contributing respectively to 60%, 30% and 3% of the intake of sterols of vegetable origin. On the contrary, the amounts of vegetable stanols in nature are much lower (mainly sitostanol and campestanol). To the purpose of the present invention a usable commercial source of phytosterols is represented by talloil (sub-product of the Kraft process for the woodworking), the percentage composition thereof is the following one: Beta-sitosterol max 80%, sitosterol max 15%, campesterol max 10%, stigmasterol max 3%, brassicasterol max 3%, campestanol max 2%, other sterols max 3%.

The role that sterols and stanols have in contributing to the reduction in high levels of cholesterol in the blood is well documented. They inhibit the absorption of cholesterol at intestinal level, most probably by means of preventing the process of intraluminal solubilisation.

In a preferred embodiment, the composition of the invention comprises, for each daily dosed amount, an amount of sterols and/or stanols comprised between 500 and 1000 mg. Preferably, the composition of the invention comprises, for each daily dosed amount, an amount of sterols and/or stanols comprised between 600 and 900 mg.

In a particular embodiment and with the purpose of obtaining an effective coadjuvant and stimulation action of the organism metabolism in relation to the antidyslipidemic action of the active ingredients in such sense (red yeast rice, long chain linear aliphatic alcohols, niacin and sterols /stanols), the composition of the invention, further comprises for each daily dosed amount an effective amount of at least a vitamin selected in the group comprising; folic acid and derivatives thereof, vitamin B6, vitamin B12 and vitamin E.

Advantageously and preferably, the composition of the invention further incorporates - for each unit dosage - at least one excipient acceptable from a dietary point of view, in particular a food excipient selected among those commonly used in the field of the formulation of the dietetic compositions and of the food integrators.

By way of example and without any limitative purpose, such excipients can be selected among those commonly used in the field such as e.g. those authorized by the Ministry of Health for the formulation of foods and enclosed to the MD 27/02/96 Nr°209.

The composition of the invention can be formulated in solid or liquid form, depending upon the nature of the various ingredients, by employing techniques and preparatory modes known to the person skilled in the art.

The composition of the invention then can be advantageously formulated in form of capsules, soft capsules, microcapsules, tablets, chewable tablets, pearls, soluble powder, granulate, syrup, gel, solution, emulsion, suspension or dispersion or oral drops by selecting in a suitable way and according to the knowledge of the person skilled in the art the various ingredients in the suitable physical form.

In a specific embodiment of the invention, the single components of the composition of the invention can be packaged separately one from the other or in a joined form in a Kit of parts, intended for simultaneous or consecutive administration. The kit of parts of the present invention is as defined in the appended claims. According to the present invention the expression "for single dosage unit" the unitary formulation is meant, for example the single capsule, tablet, pearl. When the composition is in physically not defined form, such as powder, granulate, gel, solution, emulsion, suspension or dispersion, the single dosage unit corresponds to the amount of medicament closed for example in a small bag or sachet or stick pack or other single-dose container. Alternatively, when the composition is in liquid form, the single dosage unit corresponds to the amount in ml included in a reference measuring container or corresponding to a defined number of drops.

The unitary formulation can contain an amount of composition corresponding to the minimum daily dosage. In this way the daily administration of a unitary formulation or dosage unit will correspond to the needed daily amount.

The preparation of a composition according to the present invention, however, is not an obvious task for a person skilled in the art, as it can imply complex technical problems due to the features of the active principles in the respectively set amounts and to the need of obtaining a composition pleasant and acceptable by the patient.

A preferred embodiment of the present invention is constituted by an oral composition selected among:
- a) solid composition in form of tablets
- b) composition in form of liquid suspension on aqueous base
- c) solid composition in form of chewable pastille.

A preferred embodiment of the invention is constituted by a composition in oral solid form of tablet wherein, apart from the above-mentioned active principles, excipients are used selected among i) fillers selected in the group constituted by microcrystalline cellulose, starch and calcium phosphate, ii) lubricants selected in the group constituted by magnesium stearates, calcium and zinc, and iii) glidants selected in the group constituted by silicon dioxide, starch and talcum; preferably as filler the microcrystalline cellulose can be used, as lubricant the magnesium stearate and as glidant the silicon dioxide.

Another preferred embodiment of the invention is constituted by a composition in the form of oral aqueous liquid suspension wherein, apart from the above-mentioned active principles, excipients are used selected among fillers, thickeners, moistening agents, antioxidants, emulsifying agents, preservatives, flavouring agents, dyes and sweeteners; preferably one can use: i) sorbitol as filler, moistening agent and sweetener, ii) glycerol as moistening agent, iii) acacia rubber and/or xanthan gum as thickeners, iv) the ascorbic acid as antioxidant, v) sucrose esters with fatty acids as emulsifying agents, vi) sodium benzoate and/or potassium sorbate as preservatives, vii) the raspberry flavour as flavouring agent, viii) carrot and hibiscus juice and dehydrated pulp as natural dyes, ix) sucralose as sweetener. Such composition results stable at room temperature (it does not need preservation at low temperature), it can be ingested immediately and it has optimum palatability.

An additional preferred embodiment of the invention is constituted by a composition in oral solid form of chewable tablet wherein, in addition to the above-mentioned active principles, excipients are used selected from i) lubricants selected in the group constituted by magnesium stearates, calcium and zinc, ii) glidants selected in the group constituted by silicon dioxide, starch and talcum, iii) edulcorants selected in the group constituted by mannitol, sucralose, neohesperidin and syrup of glucose iv) flavors v) hydrogenated vegetable fats such as vegetable stearin; preferably as lubricant the magnesium stearate can be used and as glidant the silicon dioxide.

All compositions implemented according to the present invention are suitable to the administration of a single dosage unit per day, but depending upon the patient's needs the administration of two or three dosage units per day is also possible.

Additional features and advantages of the invention will result more from the following description of some not limitative examples of dietetic compositions according to the invention.

### EXAMPLE 1

### Oral composition in form of tablets

Formulation of each single dosage unit:

| | |
|---|---|
| Red yeast rice * | 167 mg |
| Polycosanols** | 10 mg |
| Niacin | 35 mg |
| Sterols | 800 mg |
| Microcrystalline cellulose | 270 mg |
| Magnesium stearate | 9 mg |
| Silicon dioxide | 9 mg |
| *Total* | 1300 mg |

| | |
|---|---|
| * titre in mevinolin: 3% by weight ** polycosanols from sugar cane wax having titre in octacosanol around 60% (between 58 and 62%). | |

Preparation process: the single components of the formulation are weighed in the suitable amounts and mixed in a mixer until a perfect homogenization. The so-obtained mixture is introduced in suitable compressing apparatus to obtain, by direct compression, tablets with required weight and hardness.

### EXAMPLE 2

### Composition in form of liquid suspension

Formulation of each single dosage unit:

| | |
|---|---|
| Red yeast rice * | 168 mg |
| Polycosanols** | 10 mg |
| Niacin | 27 mg |
| Sterols*** | 890 mg |
| Glycerol | 6000 mg |
| Sorbitol | 600 mg |
| Carrot and hibiscus juice and dehydrated pulp | 120 mg |
| Acacia gum | 40 mg |
| Xanthan gum | 40 mg |
| Ascorbic acid | 35 mg |
| Aroma Lampone | 30 mg |
| Sodium benzoate + potassium sorbate (preservatives) | 38 mg (18 + 20 mg) |
| Sucrose esters of fatty acids (emulsifying agent) | 12 mg |
| Sucralose (sweeteners) | 3 mg |
| Water | as required to 15000 mg |
| *Total* | 15000 mg |

| | |
|---|---|
| * titre in mevinolin: 3% by weight ** polycosanols from sugar cane wax having titre in octacosanol around 60% (betweem 58 and 62%). *** concentrate of hydrodispersible phytosterols containing 90% of sterols, deriving from talloil | |

Preparation process: the single components of the formulation, both solids and liquids, are weighed in the suitable amounts and mixed in a mixer until a perfect homogenization of the resulting suspension in aqueous phase. The so-obtained suspension, after suitable physical checks of the half-finished product, is poured into a container for food use and from this one packed in single-dose stick pack. Once made the suitable quality checks, one proceeds with the packaging in the final cases.

The so-obtained composition is stable at room temperature (it does not need preservation at low temperature), it can be ingested immediately and it has optimum palatability.

### EXAMPLE 3

### Composition in form of chewable tablet

Formulation of each single dosage unit:

| | |
|---|---|
| Red yeast rice * | 167 mg |
| Polycosanols** | 10 mg |
| Niacin | 32 mg |
| Sterols | 800 mg |
| Mannitol | 460 mg |
| Sucralose | 5 mg |
| Neohesperidin | 1 mg |
| Syrup of glucose | 466 mg |
| Vegetable stearin | 60mg |
| Citric acid | 50 mg |
| Flavors | 13 mg |
| Silicon dioxide | 20 mg |
| Magnesium stearate | 16 mg |
| *Total* | 2100 mg |

| | |
|---|---|
| * titre in mevinolin: 3% by weight ** polycosanols from sugar cane wax having titre in octacosanol of 60%. | |

Preparation process: the single active principles composing the formulation (red yeast rice, polycosanols, niacin and sterols) are weighed in the suitable amounts and mixed with silicon dioxide and magnesium stearate in a mixer until a perfect homogenization. The so-obtained mixture is pre-compressed and granulated. The granules are sieved and passed in a rotating mixer. To the granules, kept under vigorous stirring, the vegetable stearin is added at a temperature of 70°C until a complete covering of the granules. The mass temperature is kept around 45°C during the whole covering process. The edulcorants, the citric acid and the flavors are pre-mixed separately and added to the covered granules. The so-obtained mixture is introduced in suitable compressing apparatus in order to obtain, by direct compression, tablets with required weight and hardness.

### EXAMPLE 4

### Antidvslipidemic action

In order to verify in vivo the antidyslipidemic action consequent to the administration of the dietetic compositions of the invention, the following experimental protocol was used.

Each one of the compositions of the previous Examples 1-3 was tested on a group of patients affected by hypercholesterolemia. The test admission criterium was exclusively of ethical type, that is the subjects should not have pathologies correlated to the cardiovascular system, that is they should be in primary therapy.

The protocol provided a preparatory or "RUN-IN" phase wherein the subjects were subjected (when not already in this phase) to a period of at least 15 days of stabilization diet, wherein they should follow the general suggestions related to hygienic-alimentary healthy habits (no restrictive, nor typically hypocholesterolemizing diets). Once performed the checking haematochemical examinations, the subject then, before going to bed, took 2 tablets per day of the composition to be tested for the first 30 days of treatment, which continued then with the dose of 1 tablet per day for further 30 days.

At the end of the period provided for the administration of the composition of the invention (60 days), each subject was submitted again to haematochemical check. The obtained results showed that the particular association of ingredients with antidyslipidemic action of the present invention was surprisingly capable of reducing total cholesterol in some cases up to 27%.

The remarkable improving effects of the composition of the invention - substantially comparable to those of a pharmaceutical treatment -were, at last, obtained with a substantial absence of side effects to the whole advantage of the health and welfare state of the treated subject.

Of course, the compositions shown in the above examples must be meant as a pure not limitative exemplification of some possible formulations of the composition of the invention, clearly being understood that the dosage and the specific features of the various used ingredients, such as for example the title in mevinolin of the specific used microrganism or the specific composition of the mixture of long chain aliphatic alcohols, or the specific composition of the various components existing in the sterols and/or stanols of vegetable origin, could be varied by the person skilled in the art to meet specific and contingent needs even if within what described and claimed.

## Claims

1. A composition comprising per dosage unit:
- red yeast rice having a titre in mevinolin comprised between 0.05 and 5% by weight;
- polycosanols;
- niacin;
- from 500 to 1000 mg, preferably from 600 to 900 mg of sterols and/or stanols at least one pharmaceutically acceptable excipient.

2. The composition according to claim 1 comprising per dosage unit:
- red yeast rice having a titre in mevinolin comprised between 0.05 and 5% by weight;
- polycosanols;
- from 4 to 100 mg, preferably from 10 to 50 mg of niacin; and
- sterols and/or stanols at least one pharmaceutically acceptable excipient.

3. The composition according to claims 1 or 2, comprising per dosage unit:
- red yeast rice having a titre in mevinolin comprised between 0.05 and 5% by weight;
- from 1 to 50 mg, preferably from 5 to 20 mg, of polycosanols;
- niacin; and
- sterols and/or stanols at least one pharmaceutically acceptable excipient.

4. The composition according to anyone of claims 1 to 3, comprising per dosage unit:
- from 50 to 600 mg, preferably from 100 to 300 mg, of red yeast rice having a titre in mevinolin comprised between 0.05 and 5% by weight;
- polycosanols;
- niacin; and
- sterols and/or stanols at least one pharmaceutically acceptable excipient

5. The composition according to anyone of claims 1-4, comprising per dosage unit an amount of red yeast rice adapted to make a total quantity of mevinolin comprised between 0.5 and 15 mg, preferably between 1 and 10 mg, more preferably comprised between 2 and 6 mg.

6. The composition according to claims 1-5, wherein said red yeast rice is obtained with a yeast belonging to the class *Ascomycota,* order *Eurotiales,* genus *Basipeiospora,* species *Monascus.*

7. The composition according to anyone of claims 1-6, comprising polycosanols consisting of a mixture of linear aliphatic alcohols having from 14 to 36 carbon atoms, having a titre in octacosanol comprised between 50 and 80% by weight.

8. The composition according to claim 7 comprising polycosanols consisting of a mixture of linear aliphatic alcohols wherein, in addition to ocstacosanol, the tetracosanol, the hexacosanol, the triacontanol and the dotriacontanol are present.

9. The composition according to anyone of claims 1-8, further comprising for each unit dosage an effective amount of at least one ingredient selected from the group comprising: folic acid, Vitamin B6, vitamin B12, vitamin E and a dietary acceptable excipient.

10. The composition according to anyone of the preceding claims, in solid or liquid form, selected in the group consisting of: capsules, soft capsules, microcapsules, tablets, chewable tablets, pearls, soluble powder, granulate, syrup, gel, solution, emulsion, suspension or dispersion or oral drops, even in form of bag, sachet or single-dose stick pack.

11. The composition according to claim 10, in the form of tablet, having the following formula: red yeast rice 167 mg, polycosanols 10 mg, niacin 35 mg, sterols 800 mg, microcrystalline cellulose 270 mg, magnesium stearate 9 mg, silicon dioxide 9 mg.

12. The composition according to claim 10, in the form of liquid suspension, having the following formula: red yeast rice 168 mg, polycosanols 10 mg, niacin 27 mg, sterols 890 mg, glycerol 6000 mg, sorbitol 600 mg, carrot and hibiscus juice and dehydrated pulp 120 mg, acacia gum 40 mg, xanthan gum 40 mg, ascorbic acid 35 mg, sodium benzoate plus potassium sorbate 38 mg, sucrose esters of fatty acids 12 mg, sucralose 3 mg, water as needed to 15 g.

13. The composition according to claim 10, in the form of chewable tablet, having the following formula: rice red yeast 167 mg, polycosanols 10 mg, niacin 5 32 mg, sterols 800 mg, mannitol 460 mg, sucralose 5 mg, neohesperidin 1 mg, syrup of glucose 466 mg, vegetable stearin 60mg, citric acid 50mg, flavors13 mg, silicon dioxide 20 mg, magnesium stearate 16 mg.

14. The composition according to anyone of the preceding claims, for use in the treatment or prophylaxis of dyslipidemia.

15. The composition for use according to claim 14, wherein the treatment provides daily administrations of sterols/stanols from 500 to 2000 mg/die, preferably from 500 to 1000 mg/die.

16. Kit of parts containing separately each of the components of the compositions according to anyone of claims 1 to 9, for simultaneous or consecutive administration.

## Patentansprüche

1. Zusammensetzung, die pro Dosierungseinheit umfasst:
- Rote Reis-Hefe mit einem Titer von Mevinolin zwischen 0,05 und 5 Gewichtsprozent;
- Polycosanole;
- Niacin;
- 500 bis 1000 mg, vorzugsweise 600 bis 900 mg, Sterole und/oder Stanole als mindestens einen pharmazeutisch akzeptablen Hilfsstoff.

2. Zusammensetzung gemäß Anspruch 1, die pro Dosierungseinheit umfasst:
- Rote Reis-Hefe mit einem Titer von Mevinolin zwischen 0,05 und 5 Gewichtsprozent;
- Polycosanole;
- 4 bis 100 mg, vorzugsweise 10 bis 50 mg, Niacin; und
- Sterole und/oder Stanole als mindestens einen pharmazeutisch akzeptablen Hilfsstoff.

3. Zusammensetzung gemäß den Ansprüchen 1 oder 2, die pro Dosierungseinheit umfasst:
- Rote Reis-Hefe mit einem Titer von Mevinolin zwischen 0,05 und 5 Gewichtsprozent;
- 1 bis 50 mg, vorzugsweise 5 bis 20 mg, Polycosanole;
- Niacin; und
- Sterole und/oder Stanole als mindestens einen pharmazeutisch akzeptablen Hilfsstoff.

4. Zusammensetzung gemäß einem der Ansprüche 1 bis 3, die pro Dosierungseinheit umfasst:
- 50 bis 600 mg, vorzugsweise 100 bis 300 mg, Rote Reis-Hefe mit einem Titer von Mevinolin zwischen 0,05 und 5 Gewichtsprozent;
- Polycosanole;
- Niacin; und
- Sterole und/oder Stanole als mindestens einen pharmazeutisch akzeptablen Hilfsstoff.

5. Zusammensetzung gemäß einem der Ansprüche 1-4, die pro Dosierungseinheit umfasst: eine Menge an Roter Reis-Hefe, die geeignet ist, eine Gesamtmenge an Mevinolin auszumachen, die zwischen 0,5 und 15 mg, vorzugsweise zwischen 1 und 10 mg, vorzugsweiser zwischen 2 und 6 mg beträgt.

6. Zusammensetzung gemäß den Ansprüchen 1-5, wobei die Rote Reis-Hefe mit einer Hefe erhalten wird, die zu der Klasse *Ascomycota,* Ordnung *Eurotiales,* Gattung *Basipeiospora,* Art *Monascus* gehört.

7. Zusammensetzung gemäß einem der Ansprüche 1-6, die umfasst: Polycosanole, die aus einer Mischung von linearen aliphatischen Alkoholen mit 14 bis 36 Kohlenstoffatomen bestehen, mit einem Titer von Octacosanol zwischen 50 und 80 Gewichtsprozent.

8. Zusammensetzung gemäß Anspruch 7, die umfasst: Polycosanole, die aus einer Mischung von linearen aliphatischen Alkoholen besteht, wobei, zusätzlich zu Octacosanol, das Tetracosanol, das Hexacosanol, das Triacontanol und das Dotriacontanol vorhanden sind.

9. Zusammensetzung gemäß einem der Ansprüche 1-8, die weiterhin für jede Dosierungseinheit eine wirksame Menge mindestens einer Ingredienz umfasst, die aus der folgenden Gruppe ausgewählt wird: Folsäure, Vitamin B6, Vitamin B12, Vitamin E und ein diätetisch akzeptabler Hilfsstoff.

10. Zusammensetzung gemäß einem der vorangehenden Ansprüche, in fester oder flüssiger Form, die aus der folgenden Gruppe ausgewählt wird: Kapseln, Weichkapseln, Mikrokapseln, Tabletten, Kautabletten, Perlen, lösliches Pulver, Granulat, Sirup, Gel, Lösung, Emulsion, Suspension oder Dispersion oder Tropfen zur oralen Einnahme, auch in Form einer Tüte, eines Beutels oder eines Einzeldosis-Stickpacks.

11. Zusammensetzung gemäß Anspruch 10, in der Form einer Tablette, mit der folgenden Rezeptur: Rote Reis-Hefe 167 mg, Polycosanole 10 mg, Niacin 35 mg, Sterole 800 mg, mikrokristalline Zellulose 270 mg, Magnesiumstearat 9 mg, Siliziumdioxid 9 mg.

12. Zusammensetzung gemäß Anspruch 10, in der Form einer flüssigen Suspension, mit der folgenden Rezeptur: Rote Reis-Hefe 168 mg, Polycosanole 10 mg, Niacin 27 mg, Sterole 890 mg, Glycerol 6000 mg, Sorbitol 600 mg, Karotten- und Hibiskussaft und dehydriertes Fruchtfleisch 120 mg, Gummi arabicum 40 mg, Xanthan 40 mg, Ascorbinsäure 35 mg, Natriumbenzoat plus Kaliumsorbat 38 mg, Zuckerester von Fettsäuren 12 mg, Sucralose 3 mg, Wasser nach Bedarf bis 15 g.

13. Zusammensetzung gemäß Anspruch 10, in der Form einer Kautablette, mit der folgenden Rezeptur: Rote Reis-Hefe 167 mg, Polycosanole 10 mg, Niacin 32 mg, Sterole 800 mg, Mannitol 460 mg, Sucralose 5 mg, Neohesperidin 1 mg, Glukosesaft 466 mg, pflanzliches Stearin 60 mg, Zitronensäure 50 mg, Aromen 13 mg, Siliziumdioxid 20 mg, Magnesiumstearat 16 mg.

14. Zusammensetzung gemäß einem der vorangehenden Ansprüche zur Verwendung bei der Behandlung oder Prophylaxe von Fettstoffwechselstörung.

15. Zusammensetzung zur Verwendung gemäß Anspruch 14, wobei die Behandlung tägliche Verabreichungen von Sterolen/Stanolen von 500 bis 2000 mg/Tag, vorzugsweise von 500 bis 1000 mg/Tag, zur Verfügung stellt.

16. Baukastensystem, das getrennt jede der Komponenten der Zusammensetzungen gemäß einem der Ansprüche 1 bis 9, zur gleichzeitigen oder aufeinander folgenden Verabreichung, enthält.

## Revendications

1. Composition comprenant, par dose unitaire :
- du riz de levure rouge ayant un titre de mévinoline compris entre 0,05 et 5 % en poids ;
- des polycosanols ;
- de la niacine ;
- de 500 à 1000 mg, de préférence de 600 à 900 mg de stérols et/ou stanols ;
au moins un excipient pharmaceutiquement acceptable.

2. Composition selon la revendication 1 comprenant, par dose unitaire :
- du riz de levure rouge ayant un titre de mévinoline compris entre 0,05 et 5 % en poids ;
- des polycosanols ;
- de 4 à 100 mg, de préférence de 10 à 50 mg de niacine ; et
- des stérols et/ou stanols ;
au moins un excipient pharmaceutiquement acceptable.

3. Composition selon la revendication 1 ou 2 comprenant, par dose unitaire :
- du riz de levure rouge ayant un titre de mévinoline compris entre 0,05 et 5 % en poids ;
- de 1 à 50 mg, de préférence de 5 à 20 mg de polycosanols ;
- de la niacine ; et
- des stérols et/ou stanols ;
au moins un excipient pharmaceutiquement acceptable.

4. Composition selon l'une quelconque des revendications 1 à 3 comprenant, par dose unitaire :
- de 50 à 600 mg, de préférence de 100 à 300 mg de riz de levure rouge ayant un titre de mévinoline compris entre 0,05 et 5 % en poids ;
- des polycosanols ;
- de la niacine ; et
- des stérols et/ou stanols ;
au moins un excipient pharmaceutiquement acceptable.

5. Composition selon l'une quelconque des revendications 1 à 4 comprenant, par dose unitaire, une quantité de riz de levure rouge adaptée pour que soit obtenue une quantité totale de mévinoline comprise entre 0,5 et 15 mg, de préférence entre 1 et 10 mg, mieux encore comprise entre 2 et 6 mg.

6. Composition selon l'une quelconque des revendications 1 à 5, dans laquelle ledit riz de levure rouge est obtenu avec une levure appartenant à la classe *Ascomycota,* ordre *Eurotiales,* genre *Basipeiospora,* espèce *Monascus.*

7. Composition selon l'une quelconque des revendications 1 à 6, comprenant des polycosanols consistant en un mélange d'alcools aliphatiques linéaires ayant de 14 à 36 atomes de carbone, ayant un titre d'octacosanol compris entre 50 et 80 % en poids.

8. Composition selon la revendication 7, comprenant des polycosanols consistant en un mélange d'alcools aliphatiques linéaires, dans laquelle, en plus de l'octacosanol, sont présents du tétracosanol, de l'hexacosanol, du triacontanol et du dotriacontanol.

9. Composition selon l'une quelconque des revendications 1 à 8, comprenant en outre, pour chaque dose unitaire, une quantité efficace d'au moins un ingrédient choisi dans le groupe comprenant : l'acide folique, la vitamine B6, la vitamine B12, la vitamine E, et un excipient alimentaire acceptable.

10. Composition selon l'une quelconque des revendications précédentes, sous forme solide ou liquide, choisie dans le groupe constitué par : les capsules, les capsules molles, les microcapsules, les comprimés, les tablettes à mâcher, les perles, une poudre soluble, un granulat, un sirop, un gel, une solution, une émulsion, une suspension et une dispersion et des gouttes à usage oral, même sous forme de sac, sachet, ou bâtonnet monodose.

11. Composition selon la revendication 10, sous forme de comprimé, ayant la formulation suivante: 167 mg de riz de levure rouge, 10 mg de polycosanols, 35 mg de niacine, 800 mg de stérols, 270 mg de cellulose microcristalline, 9 mg de stéarate de magnésium, 9 mg de dioxyde de silicium.

12. Composition selon la revendication 10, sous forme de suspension liquide, ayant la formulation suivante : 168 mg de riz de levure rouge, 10 mg de polycosanols, 27 mg de niacine, 890 mg de stérols, 6000 mg de glycérol, 600 mg de sorbitol, 120 mg de pulpe déshydratée et de jus de carotte et d'hibiscus, 40 mg de gomme arabique, 40 mg de gomme xanthane, 35 mg d'acide ascorbique, 38 mg de benzoate de potassium plus sorbate de potassium, 12 mg d'esters de saccharose et d'acides gras, 3 mg de sucralose, de l'eau selon les besoins pour 15 g.

13. Composition selon la revendication 10, sous forme d'une tablette à mâcher, ayant la formulation suivante: 167 mg de riz de levure rouge, 10 mg de polycosanols, 32 mg de niacine, 800 mg de stérols, 460 mg de mannitol, 5 mg de sucralose, 1 mg de néohespéridine, 466 mg de sirop de glucose, 60 mg de stéarine végétale, 50 mg d'acide citrique, 13 mg d'arômes, 20 mg de dioxyde de silicium, 16 mg de stéarate de magnésium.

14. Composition selon l'une quelconque des revendications précédentes, pour une utilisation dans le traitement ou la prophylaxie d'une dyslipidémie.

15. Composition pour une utilisation selon la revendication 14, dans laquelle le traitement assure des administrations quotidiennes de stérols/stanols de 500 à 2000 mg/jour, de préférence de 500 à 1000 mg/jour.

16. Kit de pièces contenant séparément chacun des composants des compositions selon l'une quelconque des revendications 1 à 9, pour une administration simultanée ou consécutive.
